# EUROPÄISCHE PATENTSCHRIFT

(11) **EP 2 358 363 B1**
(45) Veröffentlichungstag und Bekanntmachung des Hinweises auf die Patenterteilung: **07.09.2016**
(21) Anmeldenummer: 09771503.1
(22) Anmeldetag: 26.11.2009
(51) Int. Cl.: A61K 9/70, A61L 15/44

(54) **POLYURETHANPFLASTER FÜR DIE TRANSDERMALE APPLIKATION VON WIRKSTOFFEN UND VERFAHREN ZU DESSEN HERSTELLUNG**
TRANSDERMAL POLYURETHAN PATCH AND ITS PREPARATION
TIMBRE TRANSDERMIQUE DE POLYURÉTHANE ET PRÉPARATION

(30) Priorität: 26.11.2008 DE 102008059054
(43) Veröffentlichungstag der Anmeldung: 24.08.2011
(73) Patentinhaber: Otto Bock Pur Lifescience GmbH, 37115 Duderstadt (DE)
(72) Erfinder: GANSEN, Peter, 37136 Seeburg (DE); DITTGEN, Michael, 99510 Apolda (DE); STEINFATT-HOFFMANN, Ingeborg, 37412 Hörden (DE); SCHULTE, Christian, 37075 Göttingen (DE); HENZE, Jürgen, 37115 Duderstadt (DE)
(74) Vertreter: Lins, Martina
(86) Internationale Anmeldenummer: PCT/EP2009/008432
(87) Internationale Veröffentlichungsnummer: WO 2010/060621

(56) Entgegenhaltungen:
- EP-A1- 1 249 480
- EP-A1- 1 438 943
- EP-A1- 1 815 875
- EP-A2- 1 190 723
- WO-A1-93/10772
- WO-A2-01/39752
- DE-A1- 10 047 700
- DE-A1- 10 128 685
- DE-A1- 10 360 592
- DE-A1- 19 526 864
- DE-A1- 19 738 855
- US-A- 5 686 098

## Beschreibung

Die Erfindung betrifft ein Verfahren zur Herstellung eines wirkstoffhaltigen Polyurethanpflasters durch Reaktivbeschichtung einer Unterlage für die transdermale Applikation von Wirkstoffen sowie eine Polyurethanpflasterbahn als Halbzeug oder Rollenmaterial, die für die Portionierung zu Einzelpflastern vorgesehen ist.

In Stand der Technik sind verschiedene therapeutisch nutzbare transdermale Pflaster bekannt, bei denen lösungsmittelhaltige Polymermischungen auf eine Trägerfolie aufgebracht werden und danach das Lösungsmittel durch Trocknung entfernt wird.

So ist aus der DE 43 01 783 C1 (LTS Lohmann) ein transdermales therapeutisches System mit Galanthamin als wirksamem Bestandteil bekannt, bei dem das transdermale therapeutische System, also das wirkstoffhaltige Pflaster, den Wirkstoff Galanthamin an die Haut abgibt. Das System ist mit einer für den Wirkstoff undurchlässigen Rückschicht und einer haftklebenden Reservoirschicht ausgerüstet. Bei dem bekannten transdermalen System kann es zu Problemen mit Restlösemitteln kommen, da die vollständige Verdampfung des Lösungsmittels aus der Polymermatrix praktisch nicht möglich ist. Das System dunstet daher nach der Herstellung noch lange Zeit Lösungsmittel aus - auch auf der und durch die Haut, während der Wirkstoffabgabe - was nicht erwünscht ist. Durch die Abdunstung schwankt auch die Wirkstoffkonzentration beim Auftrag auf den Träger.

Der Versuch, die Polymerlösung, die als Matrix zur Freigabe des Wirkstoffs dienen soll, durch eine Polymerschmelze zu ersetzen, muss bei temperaturempfindlichen Wirkstoffen fehlschlagen.

Die US 5,686,098 gibt ein dreischichtiges Östrogen-Wirkstoffpflaster an, das aus einer Trägerschicht für die Wirkstoffmatrix (backing layer), einer Schicht aus der Wirkstoffmatrix selbst, die einen Haftklebstoff beinhaltet, und einer die Wirkstoffmatrix abdeckenden abziehbaren Schutzschicht (removable protective layer) besteht.

Ähnliche Wirkstoffpflaster sind in der DE 103 60 592 A1 und der WO 93/10772 angegeben. Entsprechende Pflasteraufbauten sind auch als Wundabdeckungen in Gebrauch. Die Matrix ist dann häufig eine hydrophile Polyurethanmatrix, die geschäumt sein kann, und dient zur Aufnahme des Wundexsudats. Eine solche Wundabdeckung mit aufliegender Keimbarriere und unterliegender Haftklebstoffschicht ist in der EP 1 815 875 A1 offenbart.

Die DE 101 28 685 offenbart ein selbstklebendes wirkstoffhaltiges Matrix-pflaster mit einer Matrix aus geschäumtem oder ungeschäumtem Polyurethan, die auf einem Träger aus silikonisiertem Papier aufliegt und mit einer ggf. klebstoffbeschichteten Folie abgedeckt ist. Eine kuppelförmige Portionierung der Matrix zu Einzelpflastern ist bevorzugt.

Die EP 1 190 723 A2 zeigt einen Wundverband aus einer wasserdampfdurchlässigen Polyurethanmatrix, die in einer mit Abdeckfolie ausgelegten Form unter Druck halbkonvex (linsenförmig) ausgeformt und mit Trennpapier abgedeckt wird.

Die WO 01/39752 A2 offenbart ein Pflaster, das leicht flüchtige Wirkstoffe aus einem Reservoir abgibt und dafür in ein Membransystem eingeschlossen ist. Dabei wird ein flüssig gefülltes Reservoir von zwei Folienverbunden umschlossen und eingesiegelt.

Ferner sind verschiedene kontinuierliche Verfahren zur Herstellung von Pflastern und Wundverbänden aus Folien und ggf. Folienverbunden bekannt, bei denen unterschiedliche Folientypen gehandhabt werden müssen.

Aus der EP 1 249 480 A1 ist ein Verfahren zur Herstellung eines Klebfolienstreifens mit ein- oder beidseitig haftklebend beschichteter Mittelfolie bekannt. Der Haftkleber beinhaltet keinen Wirkstoff. Die Beschichtung für Doppelklebebänder erfolgt in einem zweistufigen Verfahren, wobei zunächst die einseitige Beschichtungsmasse auf einen Trennpapierträger beschichtet und sodann mit der Mittelfolie belegt wird. Die zweite Beschichtung wird nach Zwischenreifung auf die freigelegte Rückseite der Mittelschicht aufgebracht und mit einer Trennfolie abgedeckt.

Aus der DE 100 47 700 A1 ist ein Verfahren zur Herstellung mehrschichtiger dünner Pflaster bekannt, bei dem ein nicht ausgehärtetes Polyurethan und eine Klebemasse zwischen einer Trennlage und einem Zwischenträger ausgewalzt werden, wobei die Dicke der mittleren Doppelschicht aus PU-Schicht und Haftklebemasse deutlich reduziert wird.

Die EP 1 438 943 A1 offenbart ein Verfahren zur Herstellung von Pflastern bestehend aus einer Wundauflage, die aus einem dreischichtigen Laminat gestanzt und über eine Vakuumwalze auf einem klebstoffbeschichteten Träger abgelegt wird. Das Laminat für die Wundauflage besteht aus einer Barriere-folie, dem selbstklebenden Polyurethanmaterial als Wundauflagenmatrix und einer Hitfsfolie, die die Klebemasse abdeckt und das Stanzen der Wundauflagepads erleichtert. Die Hilfsfolienstücke werden von den fixierten Wundauflagepads abgezogen, bevor die Pflasterbahn abschließend mit einer Schutzfolie abgedeckt wird.

Auch die DE 197 38 855 für ein wirkstoffhaltiges Polymermatrixpflaster, nicht mit Polyurethanmatrix, offenbart ein Herstellungsverfahren, bei dem Patches aus der haftklebenden Reservoirschicht auf eine ggf. haftklebende Rückschicht aus unidirektional elastischem Material aufgelegt werden.

Die EP 1 815 875 A1 offenbart einen Schaumstoff-Wundverband mit einer außen liegenden Keimbarriere aus einer auf die vorgefertigte Polyurethan-Schaumstofflage aufextrudierten Polyurethanfolie. Auf ihrer Rückseite kann die Schaumstofflage mit einem Haftkleber versehen sein.

Es sind weitere Verfahren zur Herstellung von Pflastern bekannt, bei denen Träger mit Haftklebemassen beschichtet werden. Aus der DE 197 55 437 C1 (Himmelsbach, Beiersdorf) ist ein Verfahren zur direkten Beschichtung eines dehnfähigen, vorzugsweise textilen Trägermaterials mit einer Haftklebermasse bekannt, bei der der Haftkleber durch einen Siebzylinder direkt auf den Träger aufgebracht wird. Die Beanspruchung des Trägers beim Auftrag führt jedoch leicht zu Schwankungen der Schichtdicke. Dies wäre gerade bei dünnen Schichten für wirkstoffhaltige Pflaster problematisch, da mit der Schichtdicke auch die Wirkstoffkonzentration je Auftragsfläche schwanken würde. Bislang kann dem Problem der Schichtdickeschwankungen nur mit Hilfe engmaschig am Produkt nachträglich durchgeführter Schichtdickemessungen begegnet werden, also einer Qualitätskontrolle, bei der gegebenenfalls ein hoher Ausschussanteil in Kauf genommen werden muss. Mit der Schichtdicke würde jedoch auch die Wirkstoffkonzentration schwanken. Für ein therapeutisches Pflaster, an das bezüglich der Dosiergenauigkeit hohe Ansprüche zu stellen sind (verschiedene Arzneimittelbücher und insbesondere U.S. Pharmakopoe), kommen die vorgenannten Pflaster und Verfahren nicht in Betracht, weil sie in der Produktion nur schwer beherrschbare Dosierungsungenauigkeiten zur Folge haben können.

Der Erfindung liegt daher die Aufgabe zugrunde, ein therapeutisch nutzbares Pflaster und ein Verfahren zu dessen Herstellung bereitzustellen, bei welchem die Nachteile im Stand der Technik vermieden werden. Weiterhin umfasst die Aufgabe, dass in dem Pflaster wenigstens ein Wirkstoff mit hoher Genauigkeit bezüglich der deklarierten Dosis enthalten sein soll, damit dieser aus dem Pflaster heraus (gleichmäßig) abgegeben und topisch oder transdermal zur Wirkung gebracht werden kann.

Die Aufgabe wird gelöst mit einem Verfahren zur Herstellung eines Polyurethanpflasters mit einer Trägerfolie und einer Schicht aus einer Polyurethanmatrix und optional weiteren Pflasterschichten, wobei das Verfahren kontinuierlich oder halbkontinuierlich mit Folienbahnen durchgeführt wird. Dieses Verfahren ist dadurch gekennzeichnet, dass eine wirkstoffhaltige Polyurethan-Trägermatrix auf einer elastischen Trägerfolie erzeugt wird, deren Elastizität größer oder gleich der des ausreagierten Matrix-Polyurethans ist, wobei die elastische Trägerfolie durch eine unterliegende, anhaftende, wenigstens mono-axial nicht-dehnbare Hilfsfolie wenigstens in Verarbeitungsrichtung des Auftrags nicht dehnbar gemacht wurde und eine unmittelbar vor oder mit dem Auftragsvorgang in einer Beschichtungseinheit gemischte lösungsmittelfreie wirkstoffhaltige Polyurethanmasse in einem Reaktivbeschichtungsschritt auf die elastische Trägerfolie beschichtet wird.

Das Verfahren beinhaltet eine Reaktivbeschichtung einer unmittelbar vor oder mit dem Auftragsvorgang in einer Beschichtungseinheit gemischten lösungsmittelfreien wirkstoffhaltigen Polyurethanmasse auf eine elastische und damit gleichzeitig dehnbare Trägerfolie, die durch eine unterliegende, anhaftende wenigstens mono-axial nicht-dehnbare Hilfsfolie wenigstens in Verarbeitungsrichtung des Auftrags unelastisch gemacht wurde.

Bei der Hilfsfolie handelt es sich nicht etwa um ein Förderband, da sich herausgestellt hat, dass die dann zwangsläufig innerhalb des Verfahrens notwendige Ablösung des Pflasters vom Band wiederum zu schwer kontrollierbaren Qualitätsschwankungen führt, wenn die Pflastermasse noch zu frisch ist. Die erfindungsgemäße Lösung sieht daher vor, dass eine gesonderte Hilfsfolie verwendet wird, die der zu beschichtenden elastischen Trägerfolie als Unterlage dient und mit dieser verbunden bleibt bis die Polyaddition praktisch vollständig abgeschlossen ist (Umsetzung bei der Urethanreaktion größer 95 %), d.h. bis ein Polymer-Endzustand erreicht wird.

Unter "Reaktivbeschichtung" wird hier verstanden, dass alle für das Polyurethan vorgesehenen Bestandteile gemischt und sofort aufgetragen werden, so dass die Polyadditionsreaktion während des Beschichtungsvorgangs und auf dem beschichteten Träger stattfindet.

Einer der Vorteile dieses Verfahrens liegt darin, dass Lösungsmittel komplett vermieden werden können, denn das Polyurethanrohstoffgemisch ist gut fließfähig und kann lösungsmittelfrei in dünner Schicht auf einen Träger aufgebracht werden. Hierfür beisitzt die in der Beschichtungseinheit zusammengeführte Polyurethan-Reaktivmasse vorzugsweise eine Viskosität im Bereich zwischen 1000 und 5000 mPas.

Verfahrenstechnisch günstig ist, dass Energie und Ausheizstrecken in der Anlage eingespart werden können. Ein weiterer Vorteil besteht darin, dass kein Lösungsmittel auswandern und mit dem Wirkstoff an den Patienten bzw. Pflasterträger abgegeben werden kann. Umwelttechnisch ist es insgesamt vorteilhaft, wenn der Gebrauch von Lösungsmitteln vermieden wird, da keine Recycling-Probleme beim Trocknen oder Ausdampfen des Lösungsmittels aufgeworfen werden. Ein weiterer Vorteil besteht darin, dass die Haftung des ausreagierten Polyurethans an der elastischen Folie, die beschichtet wurde, besonders gut ist, wenn das Polyurethan direkt auf der Folie polymerisiert wurde. Die Haftung ist wesentlich besser als wenn die elastische Folie nachträglich auf die auspolymerisierte Polyurethanmatrix aufgelegt würde. Eine auch bei mechanischer Belastung dauerhafte Verbindung zwischen Polyurethan und elastischer Folie kann durch Reaktivbeschichtung wesentlich leichter hergestellt werden.

Bevor die alle gewünschten Bestandteile umfassende Mischung für die Reaktivbeschichtung hergestellt wird, liegen alle diese Bestandteile getrennt oder auf wenigstens zwei Komponenten aufgeteilt vor, die nicht oder nur sehr langsam miteinander reagieren können und erst unmittelbar vor oder in der Beschichtungseinheit zusammengeführt werden. Wenigstens ein Polyurethanbestandteil, vorzugsweise dann die isocyanatreaktive Komponente, oder wenigstens eine der Komponenten, die in der Beschichtungseinheit zusammengeführt werden, enthält den Wirkstoff oder die Wirkstoffe. Der Wirkstoff kann auf mehrere Bestandteile aufgeteilt werden. Soweit als isocyanatreaktive Komponente ein Polyol verwendet wird, ist es besonders bevorzugt, den Wirkstoff oder die Wirkstoffe hiermit zu vermischen.

Bei dem Wirkstoff oder den Wirkstoffen handelt es sich vorzugsweise um wenigstens einen pharmazeutischen oder kosmetischen topisch oder transdermal verabreichbaren Wirkstoff, der bei Raumtemperatur fest oder flüssig ist. Es kann auch eine Mischung aus mehreren Wirkstoffen eingesetzt werden oder es werden im Gemisch mit einem oder mehreren pharmazeutischen Wirkstoffen zusätzliche nicht-pharmazeutische wirkende Bestandteile in die Matrix eingebracht, z.B. hautpflegende oder andere kosmetische Wirkstoffe. Unter einem "Wirkstoff' ist generell jede Substanz zu verstehen, die auf der Haut (topisch) oder durch die Haut (transdermal) eine bestimmte - im Allgemeinen medizinische oder kosmetische - Wirkung entfalten soll.

Welche Wirkstoffe verwendet werden, entscheidet der Fachmann. Es bestehen grundsätzlich keine Beschränkungen zur Wirkstoffauswahl, außer dass der Wirkstoff mit der Polyurethanmatrix nicht kovalent und damit nicht ortsfest binden soll und sich in Kontakt mit der Polyurethanmatrix in seiner Wirksamkeit nicht verändern soll. Ob der jeweilige Wirkstoff in diesem Sinne geeignet ist und mit welcher Kinetik der Wirkstoff aus der Matrix abgegeben wird, kann vom Fachmann durch Vorversuche geklärt werden.

Aufgrund ihrer Struktur sind Alkaloide und Steroide geeignet, aus einer Polyurethanmatrix abgegeben zu werden. Insbesondere hat sich gezeigt, dass die Geschlechtshormone (unter anderem Östrogene, insbesondere Estradiol, Gestagene, Progesteron, Androgene, insbesondere Testosteron, einschließlich identisch oder als Analoga synthetisierter Hormone/Hormonderivate) aus der reaktivbeschichteten Polyurethanmatrix abgegeben werden können. Ein beispielsweise geeigneter Wirkstoff ist Scopolamin.

Vorzugsweise besteht die isocyanatreaktive Komponente aus Polyolen, die wiederum vorzugsweise Polyester- und/oder Polyetherpolyole enthalten. Das Equivalentgewicht der Polyether- und/oder Polyesterpolyole ist vorzugsweise größer als 300 (Mg(weight) > 300).

Als Isocyanate werden in der Polyurethanmasse vorzugsweise aliphatische Isocyanate eingesetzt, besonders bevorzugt Hexamethylendiisocyanat. Das Verhältnis von Polyol zu Isocyanat wird so gewählt, dass das Polyol im Überschuss vorliegt, d.h. die Isocyanatkennzahl (auch als Isocyanatindex bezeichnet) ist in einer besonders bevorzugten Ausführungsform der Erfindung kleiner als 100, vorzugsweise kleiner als 80. Das ausreagierte Polyurethan ist dann fließfähig und dennoch formstabil.

Das ausreagierte Polyurethan ist selbst vorzugsweise haftklebend, so dass sich ein zusätzlicher Haftklebstoff zur Befestigung des Pflasters erübrigt. Dieser könne jedoch an einer beispielsweise überstehenden Abdeckschicht zusätzlich vorgesehen sein, wenn dies im Einzelfall gewünscht ist.

Das haftklebende Polyurethan, das als Matrix für die Freisetzung des darin enthaltenen Wirkstoffs oder der Wirkstoffe verwendet wird, haftet besser und vor allem dauerhafter auf der Haut als vergleichbare, bekannte Haftklebstoffe für Pflaster, z. B. auf PMMA-, StyrolButadien-Copolymer- oder Silikon-Basis.

Weiterhin ist es bevorzugt, wenn das ausreagierte wirkstoffhaltige und lösungsmittelfreie Polyurethan und die elastische Folie wasserdampfdurchlässig ausgebildet sind, vorzugsweise so wasserdampfdurchlässig wie mit der Grundrezeptur unter weitgehender Optimierung der ebenfalls erforderlichen Eigenschaften Haftklebrigkeit, Elastizität, Schmiegsamkeit, gute Wirkstoff-Freigabe möglich. Dadurch wird sichergestellt, dass das Pflaster atmet und Occlusionen der Haut, die nachteilig sein können, vermieden werden.

Die Bruchdehnung (Elongation , gemessen nach DIN EN ISO 1798 2008) des ausreagierten Polyurethans beträgt vorzugsweise > 150 %. Das ist insbesondere vorteilhaft, da bekannt ist, dass elastische transdermale Pflaster besser verträglich sind, da sie es der Haut ermöglichen, sich in natürlicher Weise zu bewegen.

Die Polyurethanmasse kann erforderlichenfalls Katalysatoren, Kettenverlängerer, Initiatoren und/oder Vernetzer sowie in der Polyurethanchemie weitere übliche Zusatz- und Hilfsstoffe enthalten. Ein besonderer Vorteil der Erfindung besteht jedoch gerade darin, dass auf Zusatz- und Hilfsstoffe weitgehend verzichtet werden kann. Generell sollten nur solche Zusatz- und Hilfsstoffe eingesetzt werden, deren Verwendung auf der Haut als unbedenklich angesehen werden kann. Mögliche Hilfsstoffe sind beispielsweise in der WO 00/45797A1 genannt, auf die hier Bezug genommen wird. Bevorzugte mögliche Zusatzstoffe sind unter anderem anorganische und organische Füllstoffe, wie zum Beispiel Silicabasierte Füllstoffe, Aluminiumoxid, Carbomere; Hautpflegemittel und/oder Tackifier, wie z.B. Terpene oder terpenhaltige Harze, insbesondere Tallharze oder Kolophoniumharze .

Bei der Hilfsfolie kann es sich um eine Trennfolie handeln. Generell ist als Hilfsfolie jede nicht-dehnbare Folie geeignet, die an der elastischen Folie lediglich anhaftet, d.h. nicht permanent klebt.

Die elastische Folie, auf die die Polyurethanbeschichtung aufgetragen wird, und die nicht-dehnbare Hilfsfolie sollen sich ohne mechanische Schädigung einer der Folien und vorzugsweise ohne jede Veränderung der Einzelfolien im Hinblick auf bleibende Dehnung, optische Veränderung oder Eigenschaftsveränderung irgendeiner Art, voneinander trennen, d.h. spalten lassen. Vorzugsweise lassen sich die aufeinandergelegten Folien (elastische und nicht-dehnbare Folie) oder die in einem vorgelagerten Verfahrensschritt aufeinander gepressten Folien von Hand spalten.

Das Verfahren wird kontinuierlich oder halbkontinuierlich mit Folienbahnen durchgeführt, wobei die Folienbahnen aus elastischer und nicht-dehnbarer Folie vorzugsweise bereits zuvor - in einem vorgelagerten Verfahrensschritt innerhalb derselben Produktionsanlage oder als zugeliefertes Vorprodukt - zu einer Doppelbahn zusammengeführt worden sind, die danach der Beschichtungseinheit zugeführt wird.

In der Praxis können die elastischeTrägerfolie und die nicht-dehnbare Hilfsfolie der Beschichtungseinheit getrennt, z. B. von je einer Rolle, zugeführt und unmittelbar vor der Beschichtungseinheit zusammengeführt, d.h. aufeinandergelegt oder -gepresst werden. Die elastische und die nicht-dehnbare Folie können jedoch auch in einem vorgelagerten Verfahrensschritt aufeinandergelegt oder -gepresst und dann gemeinsam aufgewickelt werden. Dieser lose, leicht spaltbare Verbund aus zwei Folien kann dann, beispielsweise von einer Rolle ablaufend, der Beschichtungseinheit zugeführt werden. Die Folien - elastische und nicht-dehnbare Folie - können auch als Verbundfolie eingesetzt werden, wobei der Verbund, beispielsweise durch punktweise Klebstoffkaschierung, besonders leicht trennbar, d.h. vorzugsweise mit der Hand spaltbar ist.

Jeder der beiden Folien, die elastische und dehnbare mit der wirkstoffhaltigen Schicht aus der Polyurethan-Trägermatrix verbundene Folie und die nicht-dehnbare unterliegende (Hilfs-) Folie, kann für sich eine Mehrschichtfolie aus coextrudierten oder kaschierten Lagen sein.

Die nicht-dehnbare Folie hat die Funktion, jede Dehnung der elastischen Folie, die als Trägerfolie für die wirkstoffhaltige Polyurethanmatrix dient und mit dieser dauerhaft verbunden sein soll, während des Beschichtungsvorgangs wenigstens in Verarbeitungsrichtung zu verhindern, um während der Verarbeitung eine mechanische Beanspruchung der elastischen Folie (in erster Linie durch Zugkräfte) und damit Schichtdickenschwankungen und Wirkstoffkonzentrationsschwankungen in der Beschichtung zu vermeiden.

Solange die nicht-dehnbare Folie mit der elastischen verbunden ist, d.h. vor der Spaltung der Folien von Hand durch den Pflasterbenutzer oder ggf. vor weiteren Verarbeitungsschritten, macht sie den gesamten Verbund aus elastischer Folie und Beschichtung unelastisch und nicht-dehnbar, und zwar wenigstens in Verarbeitungsrichtung. Hierfür kann gegebenenfalls eine nur mono-axial nicht-dehnbare Folie verwendet werden, die in Verarbeitungsrichtung, nämlich in Längsrichtung der Folienbahn nicht-dehnbar, quer hierzu, in der auf Zug nicht belasteten Richtung, jedoch dehnbar ist. Unter einer nicht-dehnbaren Folie wird daher hier immer eine "wenigstens in Verarbeitungsrichtung" nicht-dehnbare, d. h. eine wenigstens mono-axial in Verarbeitungsrichtung oder alternativ isotrop nichtdehnbare Folie verstanden.

"In Verarbeitungsrichtung" bedeutet hier bei kontinuierlicher Verfahrensführung in Richtung des Folien- und/oder Materialvorschubs. Wenn die wirkstoffhaltige Polyurethanmasse bei diskontinuierlicher Verfahrensführung beispielsweise sternförmig auf einer Fläche verteilt wird, so dass keine einheitliche Verarbeitungsrichtung vorhanden ist, kann auch keine nur mono-axial nicht-dehnbare Hilfsfolie verwendet werden. In diesem Falle wäre eine isotrop nicht-dehnbare Folie zu verwenden.

Die Nichtdehnbarkeit des gesamten Verbundes gewährleistet, dass die Dosis des Wirkstoffs pro Flächeneinheit konstant bleibt. Die nicht-dehnbare Folie soll deshalb während aller Verarbeitungsschritte, die Zug auf den Verbund bringen oder bringen könnten, mit der elastischen Trägerfolie verbunden bleiben. Wird auf die Polyurethan-Beschichtung eine Abdeckfolie bzw. Trennfolie aufgelegt, kann auch diese vorzugsweise nicht-dehnbar sein. Die Abdeckfolie kann dann die nicht-dehnbare Hilfsfolie ersetzen und der Verbund bleibt für weitere Verarbeitungsschritte insgesamt nicht-dehnbar.

Eine mögliche Verarbeitungsschrittfolge ist daher: Beschichten der Trägerfolie, die auf der Hilfsfolie aufliegt - Aufbringen einer Abdeckfolie/Trennfolie auf die Beschichtung - Spaltung von beschichteter elastischer Trägerfolie und nicht-dehnbarer Hilfsfolie und Abziehen der Hilfsfolie - Aufwickeln des Verbundes: Abdeckfolie - Beschichtung - Trägerfolie für ein Rollen-Zwischenprodukt (Pflasterbahn) oder direkte Weiterverarbeitung des Verbundes zu Einzelpflastern.

Sofern keine nicht-dehnbare Abdeck- oder Trennfolie auf die PU-Beschichtung aufgelegt wird, soll die Pflasterbahn nach Abziehen der Hilfsfolie nicht mehr mit Zug belastet werden, ein Stanzen zu Einzelpflastern bleibt jedoch möglich.

Als "nicht-dehnbare" Folien können kommerziell erhältliche Folien eingesetzt werden, die als "nicht-dehnbar" deklariert sind. Unter einer "nicht-dehnbaren" Folie wird im Rahmen dieser Erfindung eine solche verstanden, die für eine Längung um 10% eine Kraft von mehr als 3 Newton je cm Bahnbreite (> 3 N/cm) erfordert, bevorzugt eine Kraft von mehr als 5 Newton je cm Bahnbreite (> 5 N/cm). Auf die Dicke der Hilfsfolie kommt es während des Polyurethanauftrags bei der Verarbeitung nicht an.

Als nicht-dehnbare Folien für die Unterlage- oder Hilfsfolie, kommen insbesondere in Frage: nicht-dehnbare Folien aus Polyolefin, z. B. aus Polyethylen oder Polypropylen, aus nicht-dehnbarem Polyurethan, aus Polyester, Polyamid oder Polycarbonat. Die nicht-dehnbare Folie kann vorzugsweise antihaftbeschichtet sein oder plasma- oder coronabehandelt.

Als "elastische" Folien können ebenfalls kommerziell erhältliche Folien eingesetzt werden, die als solche deklariert sind. Grundsätzlich soll die Elastizität der elastischen Trägerfolie der Elastizität des ausreagierten Matrix-Polyurethans entsprechen, d.h. größer oder gleich dieser sein.

Als elastische Trägerfolien kommen insbesondere in Frage: elastische PU-Folien, andere elastische Polymerfolien, textile Träger und allgemein atmungsaktive Pflasterdeckmaterialien, wie sie dem Fachmann bekannt sind. Die elastische Folie ist vorzugsweise 10 µm bis 50 µm dick.

Für die Beschichtungseinheit sind verschiedene Ausführungsmöglichkeiten gegeben. Grundsätzlich können alle Beschichtungsanlagen verwendet werden, mit denen die Beschichtung eines Trägers in dünner Schicht, maximal im Millimeterbereich, mit flüssigviskosem Material möglich ist.

Eine bevorzugte Ausführungsform sieht vor, dass die Beschichtungseinheit in einen Schlitz ausläuft und die wirkstoffhaltige nicht ausreagierte Polyurethanmasse in dünner Schicht auf die elastische Folie beschichtet wird. Dies kann geschehen, indem die Folie unter der räumlich feststehenden Beschichtungseinheit durchgeführt wird (für kontinuierliche Verfahrensführung besonders geeigneter Auftrag) oder indem eine verfahrbare Beschichtungseinheit über einen feststehenden Tisch mit der Folie geführt wird (für diskontinuierliche Verfahrensführung besonders geeigneter Auftrag).

Bei einer alternativen und ebenfalls vorteilhaften Ausführungsform wird die wirkstoffhaltige nicht ausreagierte Polyurethanmasse mit einer Dosier- und Mischmaschine aufgetragen und mit einem Streichmesser (auch als Rakel bezeichnet) auf der elastischen Folie verteilt.

Eine andere vorteilhafte Ausführungsform besteht darin, die wirkstoffhaltige nicht ausreagierte Polyurethanmasse mit einer Dosier- und Mischmaschine aufzutragen und durch aufdrücken einer Walze zu verteilen.

Eine weitere bevorzugte Ausführungsform besteht darin, dass die reagierende Mischung in einen Trog eingetragen wird und durch Löcher/Düsen rechenartig unten aus dem Trog austritt bzw. durch viele nebeneinander angeordnete Düsen ebenfalls rechenartig auf die elastische Folie aufgebracht wird.

Während des Beschichtungsverfahrens werden die Folien bzw. der Verbund aus Folien oder beschichteten Folien über oder zwischen Rollen und bevorzugt streckenweise über einen Tisch geführt. Die Beschichtung erfolgt vorzugsweise auf dem Tisch, am Anfang des Tisches oder vor dem Tisch auf einer vorzugsweise starren Rolle.

In Weiterbildung der Erfindung kann auf die noch nicht ausreagierte Polyurethanmasse eine zusätzliche Trennfolie aufgelegt und die Masse zwischen den Folien durch Aufdrücken mit einer Walze verteilt werden, wobei die zusätzliche Trennfolie vorzugsweise von der Walze abläuft.

Die Trennfolie soll nach Ausreagieren der Polyurethanmasse, spätestens bei Gebrauch des Pflasters, leicht abziehbar sein. Für Trennfolien stehen dem Fachmann zahlreiche geeignete Materialien zur Verfügung. Für diesen Zweck sind beispielsweise im Stand der Technik silikonisierte Folien und andere Antihaftfolien, einschließlich behandelter oder beschichteter Trennpapiere im Gebrauch. Diese Trennfolien können auch hier verwendet werden. Die Trennfolie bildet gleichzeitig eine Abdeckfolie für die PU-Beschichtung und kann wie oben beschrieben ebenfalls nicht-dehnbar im Sinne der Erfindung sein.

Die Beschichtung, d.h. die Schicht aus der lösungsmittelfreien wirkstoffhaltigen Polyurethanmatrix, die nach Ausreagieren der Polyurethanmasse auf der elastischen Trägerfolie entstanden ist, besitzt vorzugsweise eine Schichtdicke kleiner oder gleich 1000 µm. Weiter vorzugsweise erfolgt die Beschichtung mit einer Schichtdicke von kleiner oder gleich 500 µm, insbesondere kleiner oder gleich 300 µm, weiter vorzugsweise zwischen 5 und 300 µm, noch mehr bevorzugt zwischen 10 und 300 µm und besonders bevorzugt zwischen 100 und 250 µm.

Es ist sehr bevorzugt, wenn innerhalb des reagierenden Polyurethans eine Temperatur von 79 °C nicht überschritten wird, vorzugsweise 55 °C nicht überschritten wird und die Temperatur insbesondere während des gesamten Verfahrens zwischen Raumtemperatur und 50 °C bleibt.

Gemäß einer bevorzugten Ausführungsform ist weiterhin vorgesehen, dass die elastische Folie und die nicht-dehnbare Folie in einem Verfahrensschritt nach Fertigstellen der Beschichtung gespalten werden und die nicht-dehnbare Folie von der beschichteten elastischen Folie abgezogen wird. Nach diesem Verfahrensschritt kann die nicht-dehnbare Folie, die nur für den Beschichtungsvorgang und ggf. als Applikationsfolie benötigt wird, durch eine andere Pflasterabdeckung ersetzt werden, d.h. anstelle der abgezogenen nicht-dehnbaren Folie kann wenigstens eine weitere Pflasterschicht aufgebracht werden. Die weiteren Pflasterschichten können einen zusätzlichen Pflasterklebstoff umfassen, der wenigstens an einem überstehenden Randbereich der zusätzlichen Schichten aufgebracht sein kann. Als oberste Abdeckschicht kann eine textile und/oder eine hautfarbene Schicht vorgesehen sein.

Insbesondere bei der kontinuierlichen Verfahrensführung auf Folienbahnen ist vorzugsweise vorgesehen, dass die beschichtete Folie in einem Verfahrensschritt nach Fertigstellen der Beschichtung und Ausreagieren des Polyurethans geschnitten und dadurch zu Pflasterabschnitten portioniert wird, vorzugsweise einschließlich der unterliegenden nicht-dehnbaren Folie oder der weiteren Pflasterschicht(en).

Ist bei einer schmalen, in Pflasterbreite vorgegebenen Folienbahn die Folienbahn der nicht-dehnbaren Hilfsfolie breiter gewählt als die der elastischen Folie mit aufliegender Polyurethanmatrix, bildet die nicht-dehnbare Folie nach dem Schneiden in Pflasterabschnitte mit definierter Dosierung einen Überstand. Die nicht-dehnbare Folie kann dann als Applikationshilfe zum Aufbringen auf die Haut verwendet werden, der Überstand erleichtert das Abziehen. In diesem Fall werden die Trägerfolie und die Hilfsfolie, d.h. die elastische Folie und die nicht-dehnbare Folie erst am Anwendungsort des Pflasters, nämlich auf der Haut des Pflasterträgers bzw. Patienten gespalten.

Zur Lösung der Aufgabe umfasst die Erfindung weiterhin eine Polyurethanpflasterbahn für die Portionierung zu Einzelpflastern, die mit dem erfindungsgemäßen Verfahren erhältlich ist, gekennzeichnet durch das Vorhandensein folgender Schichten:
- eine für den direkten Hautkontakt vorgesehene lösungsmittelfreie Schicht aus haftklebendem Polyurethan mit definierter Wirkstoffkonzentration je Flächeneinheit, wobei die Bruchdehnung des Polyurethans mehr als 150 % gemessen nach DIN EN ISO 17982008 beträgt,
- eine mit vorgenannter Schicht in Flächenkontakt stehende elastische Trägerfolie deren Elastizität größer oder gleich der des ausreagierten Matrix-Polyurethans ist,
- eine mit der elastischen Trägerfolie in Flächenkontakt stehende, anhaftende, nicht-dehnbare Folie, die eine solche Folie ist für deren Längung um 10 % eine Kraft von mehr als 3 Newton je Zentimeter Bahnbreite erforderlich ist,
- und optional eine mit der Schicht aus haftklebendem Polyurethan in Kontakt stehende nicht-dehnbare Trenn- oder Abdeckfolie.

Zusätzlich kann dabei die wirkstoffhaltige PU-Schicht mit einer Abdeck- oder Trennfolie abgedeckt sein. Diese dient als Trennfolie für das Rollenmaterial und/oder als Schutzfolie für das wirkstoffhaltige PU. Die Abdeck- oder Trennfolie, die eine silikonisierte oder sonstige Antihaftfolie oder ein Trennpapier sein kann, wird entweder vor der Konfektionierung zu Pflastern oder spätestens vom Benutzer des Pflasters vor Aufbringen auf die Haut entfernt.

Aus der oben beschriebenen Pflasterbahn, die bei kontinuierlicher Verfahrensführung als unmittelbares Verfahrensprodukt entsteht, können Polyurethanpflaster in gewünschter Form geschnitten oder gestanzt werden. Das Polyurethanpflaster ist dann ein Einzelpflaster, entstanden durch Portionierung der Pflasterbahn, welches gekennzeichnet ist durch:
- eine für den direkten Hautkontakt vorgesehene lösungsmittelfreie Schicht aus haftklebendem Polyurethan mit definierter Wirkstoffkonzentration,
- eine auf der hautabgewandten Seite der Polyurethanschicht direkt aufliegende und diese vollständig überdeckende elastische Trägerfolie,
- eine die elastische Folie vollständig überdeckende und in direktem Kontakt mit dieser stehende Abdeckung und/oder eine mit der Schicht aus haftklebendem PU in Kontakt befindliche Abdeck- oder Trennfolie, die von der PU-Schicht rückstandsfrei abgezogen werden kann.

Die Abdeckung besteht entweder aus der nicht-dehnbaren Folie oder vorzugsweise, wie oben schon beschrieben, aus wenigstens einer weiteren Schicht oder Folie, wobei alle weiteren Schichten vorzugsweise elastisch sind. Diese weiteren Schichten werden aufgebracht, nachdem die nicht-dehnbare Hilfsfolie von dem Grundpflaster aus elastischer Folie und Polyurethanmatrix abgezogen wurde.

Gemäß einer alternativen vorteilhaften Ausgestaltung besteht die Abdeckung des Polyurethanpflasters aus einer wenigstens mono-axial nicht-dehnbarer Folie, die von der elastischen Folie von Hand spaltbar ist. Die nicht-dehnbare Folie, die als Unterlage für das Beschichtungsverfahren dient, braucht in diesem Fall nicht ersetzt zu werden.

Die Abdeckung des Pflasters besitzt oder bildet vorzugsweise einen Überstand, der das Grundpflaster aus elastischer Folie und damit fest verbundener Polyurethanauflage - nämlich der wirkstoffhaltigen Matrix - schützt. Bei Bedarf kann das Pflaster an diesem Überstand leichter ergriffen und sowohl ohne Berührung der Polyurethanschicht besser appliziert als auch später von der Haut wieder abgezogen werden.

In Weiterbildung der Erfindung liegt auf der lösungsmittelfreien wirkstoffhaltigen Schicht aus Polyurethan eine zusätzliche Trennfolie auf, die von der Polyurethanschicht rückstandsfrei abgezogen werden kann.

Die Polyurethanschicht des Pflasters, die die Matrix für die Abgabe des wenigstens einen Wirkstoffs bildet ist haftklebend und schmiegsam. Der für den Übergang des Wirkstoffs vom Pflaster auf den Pflasterträger, nämlich auf bzw. durch die Haut, erforderliche innige Kontakt kann daher mit Hilfe der Polyurethan-Abgabematrix (delivery system) besonders gut hergestellt werden. Die Haftklebrigkeit erübrigt zusätzliche Maßnahmen, um das Pflaster auf der Haut des Pflasterträgers zu befestigen.

Außer topischen können insbesondere für die transdermale Applikation geeignete Wirkstoffe mit dem erfindungsgemäßen Pflaster optimal zum Einsatz gebracht werden. Durch Variation der Schichtdicke kann die Depotwirkung eingestellt werden. Die Eigenschaften des Polyurethans mit einer Isocyanatkennzahl kleiner 100 ermöglichen die Verteilung und allmähliche Auswanderung des Wirkstoffs.

Durch das Herstellungsverfahren wird sichergestellt, dass das erfindungsgemäße Pflaster den Wirkstoff in einer Dosis enthält, die weniger als ±5 %, vorzugsweise ±1 % oder geringer von der deklarierten Dosis abweicht. Dies ist ein besonderer Vorteil, der das Pflaster für viele hochwirksame Therapeutika erst einsetzbar macht. Bei Schwankungen der Schichtdicke, die im Stand der Technik durch Direktbeschichtung einer elastischen Folie entstehen konnten, kann die tatsächlich im einzelnen Pflaster vorhandene Dosis nicht hinreichend genau angegeben werden, was therapeutisch in vielen Fällen nicht tragbar ist.

Die Schmiegsamkeit des Pflasters und die gute dauerhafte Haftung garantieren einen innigen Kontakt mit der Haut über die vorgesehene Zeit der Anwendung bis zu Wochen oder Monaten.

Im Folgenden wird die Erfindung anhand von Zeichnungen näher erläutert, die jedoch ausschließlich zur nicht beschränkenden Illustration der Erfindung dienen sollen. Es zeigen:
- Fig. 1: die Herstellung einer Doppelbahn aus Träger- und Hilfsfolie in vereinfacht schematischer Querschnittsdarstellung;
- Fig. 2: die Beschichtung der Doppelbahn mit reagierender PU-Masse in entsprechender Darstellung;
- Fig. 3: ein gestanztes Pflaster,
- Fig. 3a: - im Querschnitt vor Entfernung der Hilfsfolie,
- Fig. 3b: - in Draufsicht,
- Fig. 3c: - im Querschnitt ohne Hilfsfolie mit Umverpackung
- Fig. 4: - ein weiteres Ausführungsbeispiel für die Beschichtung einer Doppelbahn mit anschließendem Auflegen einer Trenn- und Abdeckfolie
- Fig.5: - Higuchi-Plot für Wirkstofffreisetzung (Scopolamin)
- Fig.6: - Higuchi-Plot für Wirkstofffreisetzung (Testosteron)

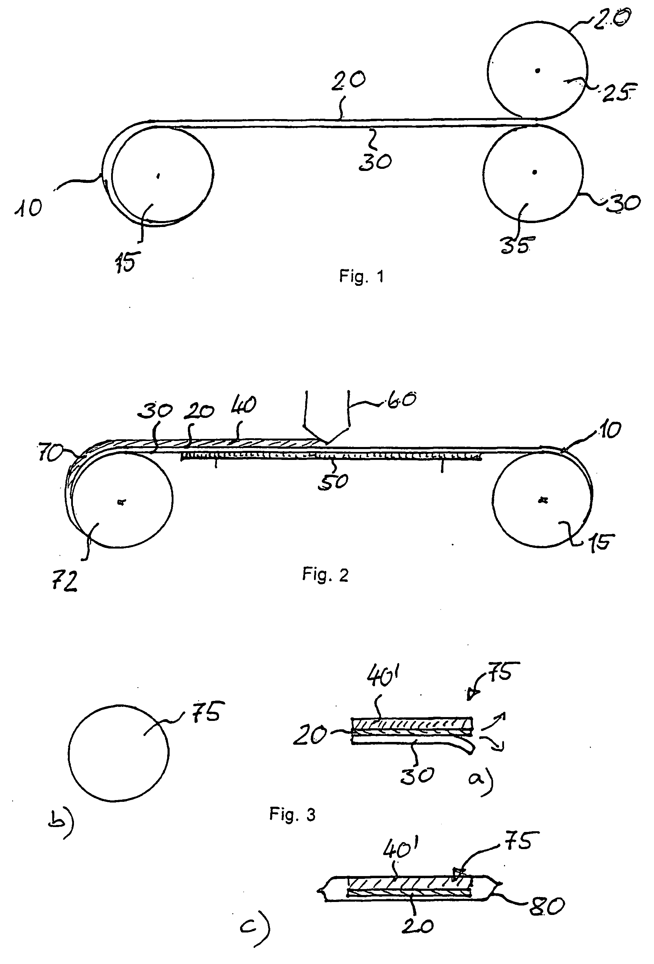

Figur 1 zeigt die Herstellung einer Doppelbahn 10 aus einer elastischen Trägerfolie 20 und einer nicht-dehnbaren Hilfsfolie 30. Beide Folien 20 und 30 werden von Rollen 25 und 35 ablaufend gegenläufig zusammengeführt. Die durch Aufeinanderlegen der Folien 20 und 30 entstehende Doppelbahn 10 wird auf eine Rolle 15 aufgerollt.

Die Haftreibung zwischen den beiden Folien 20 und 30 ist im Allgemeinen ausreichend, dass sich die beiden Folien während der nachfolgenden Beschichtung der Doppelbahn 10 nicht gegeneinander verschieben. In anderen Ausführungsbeispielen kann vorgesehen sein, dass die Folien 20 und 30 vorzugsweise punktuell mit Haftklebstoff aufeinander kaschiert werden. Sie sollen sich dabei weiterhin später von Hand spalten, d.h. voneinander trennen lassen.

In Figur 2 ist schematisch im Querschnitt gezeigt, wie die Doppelbahn 10 aus Figur 1 mit einer wirkstoffhaltigen Polyurethanmasse 40 beschichtet wird. Die Doppelbahn 10 läuft von der Rolle 15 ab und wird so über einen Tisch 50 geführt, dass die nicht-dehnbare Hilfsfolie 30 über den Tisch gleitet und dabei als unterstützende nicht-dehnbare Unterlage für die elastische Folie 20 dient. Die elastische Folie 20 wiederum bildet eine Trägerfolie für die noch reaktive Polyurethanmasse 40, die eine Matrix für einen darin enthaltenen Wirkstoff darstellt und die mit Hilfe der Beschichtungseinheit 60 während ihres Ausreagierens auf die elastische Trägerfolie 20 der Doppelbahn 10 aufgetragen wird. Die Beschichtungseinheit 60 wird getrennt mit den wenigstens zwei Komponenten für die Polyurethanreaktion gespeist (nicht dargestellt). Nachdem der Beschichtungsvorgang abgeschlossen und das Polyurethan weitgehend ausreagiert ist, wird die fertig gestellte Pflasterbahn 70 auf eine Rolle 72 gewickelt.

Figur 3 zeigt ein aus der Pflasterbahn 70 in diesem Beispiel kreisförmig gestanztes Pflaster 75. Figur 3 a) zeigt das Pflaster 75 im Querschnitt mit den Schichten Polyurethanmasse 40' (wirkstoffhaltig, ausreagiert), elastische Trägerfolie 20 und nicht-dehnbare Hilfsfolie 30. Die Folien 20 und 30 sind spaltbar, wie durch die Pfeile in Figur 3 a) angedeutet.

Figur 3 b) zeigt das Pflaster 75 in Draufsicht. Figur 3c) zeigt das Pflaster aus Figuren 3 a) und 3b) nach Abspalten der nicht-dehnbaren Hilfsfolie 30 in einer Umverpackung 80 aus Folie oder Papier. Die Polyurethanmasse 40' ist haftklebend und haftet an der Innenseite der Umverpackung 80, lässt sich jedoch von dort leicht abziehen, was erst unmittelbar vor Benutzung des Pflasters geschehen sollte. Bei Anwendung des Pflasters auf der Haut eines Benutzers wird die Polyurethanmasse 40', die den Wirkstoff enthält, durch die Trägerfolie 20 abgedeckt. Anders als in diesem Beispiel gezeigt, kann auf der Trägerfolie noch eine Pflasterabdeckung aufliegen. Die Pflasterabdeckung besteht dann entweder aus der wenigstens mono-axial nicht-dehnbaren Hilfsfolie 30 oder einer oder mehreren die Hilfsfolie 30 ersetzenden Folie(n).

Selbstverständlich kann in Abwandlung der im Beispiel gezeigten Darstellung eine Umverpackung auch mehr als ein Pflaster 75 enthalten. Für mehrere Pflaster 75 kann die Umverpackung 80 auch kompartimentiert sein.

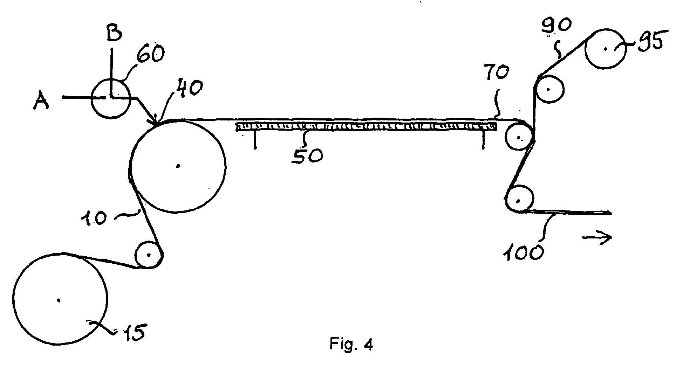

Figur 4 zeigt ein weiteres Ausführungsbeispiel für die Beschichtung einer Doppelbahn aus elastischer Trägerfolie 20 und nicht-dehnbarer Hilfsfolie 30. Die Doppelbahn 10 wird von einer Rolle 15 ablaufend über eine starre Rolle dem Tisch 50 zugeführt und auf der starren Rolle beschichtet. In der Beschichtungseinheit 60 werden die Polyurethan-Komponenten A und B zusammengeführt und aufgetragen. Die frische reaktive Polyurethanmasse 40 reagiert aus, während sie über den Tisch 50 geführt wird. Hinter dem Tisch wird die fertige Bahn 70 aus Doppelfolie und Beschichtung mit einer von einer Rolle 95 ablaufenden Abdeckfolie 90 zusammengeführt. Das Produkt 100 aus Abdeckfolie 90 und beschichteter Doppelbahn wird in Pfeilrichtung der Weiterverarbeitung zugeführt und in diesem Beispiel zu Rollenmaterial aufgewickelt, nachdem die nicht-dehnbare Folie 30 abgezogen wurde.

Die Eigenschaften der erhaltenen Pflaster wurden wie folgt geprüft:

### Beispiel 1

Transdermalpflaster (TP) mit Scopolamin

### Herstellung:

Eine Mischung aus Polyol, das 25 mg/g Scopolamin enthielt, und Polyisocyanat wurde nach dem erfindungsgemäßen Verfahren auf eine Trägerfolie von 300 µm Dicke aufgetragen. Aus der Folie wurden kreisrunde Transdermalpflaster (TP-1) mit einer Fläche von 5 cm² ausgestanzt.
Analog wurden TP mit 2,5 mg Scopolamin /g Polyol hergestellt (TP-2).

### Prüfung:

Jeweils 2 Pflaster TP-1 und TP-2 wurden vergleichend zu jeweils 2 marktüblichen TP mit Scopolamin (TP-V: Scopoderm TTS, Fa Novartis, Charge D613778, 3 mg Scopolamin/5 cm²) hinsichtlich der Wirkstofffreigabe wie folgt untersucht.
Methode: "Paddle over disk method" (USP apparatus 5 / PhEur 2.9.4.1)
Medium: Wasser mit einer Temperatur von 32°C ± 1°C
Rührgeschwindigkeit: 100 U/min
Analytik: HPLC

### Ergebnisse:

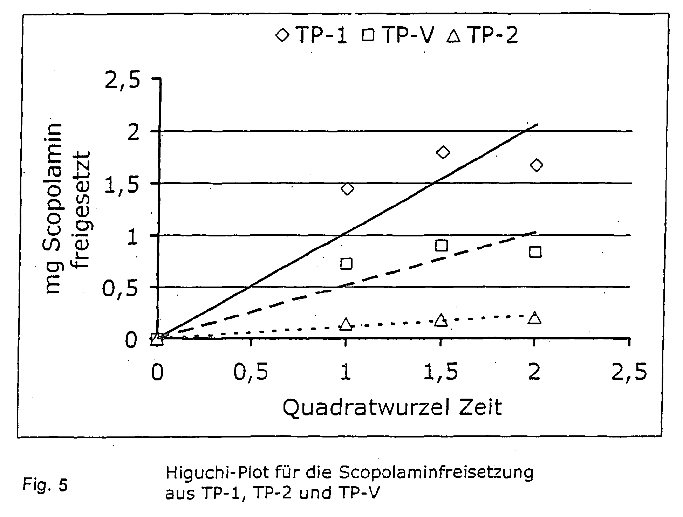

Die geprüften Pflaster setzen das enthaltene Scopolamin analog eines Wurzel-t-Matrix-Diffusions-Mechanismus frei (Higuchi-Plot, Fig. 5).

### Diskussion:

Die Messergebnisse korrelieren mit dem angenommenen Wurzel-t-Matrix-Diffusions-Mechanismus (r>0,9). Der Anstieg der Regressionsgeraden im Higuchi-Plot (Fig. 5), der die Geschwindigkeit der Wirkstofffreisetzung darstellt, beträgt
1,03 bei TP-1,
0,52 bei TP-V und
0,11 bei TP-2.

Im vorliegenden Beispiel kann die Geschwindigkeit der Wirkstofffreisetzung aus den erfindungsgemäßen TP durch die Wirkstoffkonzentration im Pflaster gesteuert werden.

Die erfindungsgemäß hergestellten Pflaster mit der höheren Wirkstoffkonzentration setzen Scopolamin schneller frei als das Vergleichspflaster. Die erfindungsgemäß hergestellten Pflaster mit der niedrigen Wirkstoffkonzentration setzen Scopolamin langsamer frei als das Vergleichspflaster. Somit besteht die Möglichkeit, durch geeignete Anpassung der Wirkstoffkonzentration eine mit dem Vergleichspräparat vergleichbare Freisetzungsgeschwindigkeit einzustellen.

### Beispiel 2

Transdermalpflaster (TP) mit Testosteron

### Herstellung:

Eine Mischung aus Polyol mit 5 % Testosteron und Polyisocyanat wurde nach dem erfindungsgemäßen Verfahren in verschiedener Schichtdicke (s) auf eine Trägerfolie aufgetragen. Aus der Folie wurden kreisrunde Transdermalpflaster mit einer Fläche von 8,4 cm² ausgestanzt.
TP-100 (s=100 µm), TP-200 (s=200 µm), TP-300 (s= 300 µm),
TP-400 (s=400 µm).

### Prüfung:

Die Pflaster TP-100 bis TP-400 wurden vergleichend zu marktüblichen TP mit Testosteron (Intrinsa: Intrinsa 300 µm/24 Stunden transdermales Pflaster, Fa. Procter & Gamble Pharmaceuticals, 8,4 mg Testosteron / 28cm², sowie Testopatch: Testopatch Transdermales Pflaster 1,8 mg/24 h, 129 cm², Fa. Pierre Fabre Pharma GmbH, Charge 7/04368/7) hinsichtlich der Wirkstofffreigabe untersucht. Dazu wurden aus den marktüblichen TP kreisrunde Stücke mit einer Fläche von 8,4 cm² ausgestanzt.
Methode: "Paddle over disk method" (USP apparatus 5 / PhEur 2.9.4.1)
Medium: Wasser mit einer Temperatur von 32°C ± 1°C
Rührgeschwindigkeit: 100 U/min
Analytik: HPLC

### Diskussion:

Die Messergebnisse korrelieren anfänglich mit dem angenommenen Wurzel-t-Matrix-Diffusions-Mechanismus (r>0,9). Bei Erschöpfung des Wirkstoffgehalts in den Pflastern endet die Korrelation.

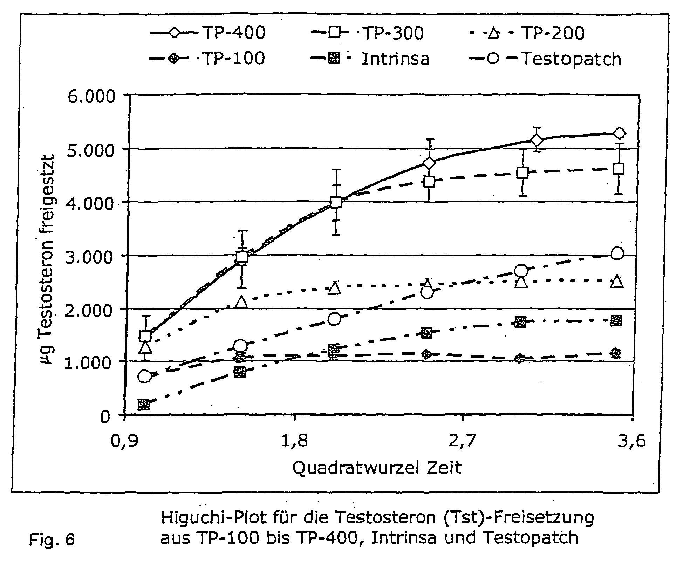

Der Anstieg der Regressionsgeraden im Higuchi-Plot (Fig. 6), der die Geschwindigkeit der Wirkstofffreisetzung darstellt, liegt für Testopatch zwischen der Geschwindigkeit der Freisetzung des Testosteron aus den erfindungsgemäßen Pflastern mit der Schichtdicke von 100 und 300 µm und entspricht etwa der Freisetzungsgeschwindigkeit beim Vergleichspräparat Intrinsa.

Im vorliegenden Beispiel kann die Geschwindigkeit der Wirkstofffreisetzung aus den erfindungsgemäßen TP durch die auf die Trägerfolie aufgetragene Schichtdicke gesteuert werden.

Die erfindungsgemäß hergestellten Pflaster mit der höheren Schichtdicke setzen Testosteron schneller frei als die Vergleichspflaster. Die erfindungsgemäß hergestellten Pflaster mit der niedrigen Schichtdicke setzen Testosteron langsamer frei als die Vergleichspflaster. Somit besteht die Möglichkeit, durch geeignete Anpassung der Auftragsschichtdicke eine mit den Vergleichspräparaten vergleichbare Freisetzungsgeschwindigkeit einzustellen.

## Patentansprüche

1. Verfahren zur Herstellung eines Polyurethanpflasters mit einer Trägerfolie und einer Schicht aus einer Polyurethanmatrix und optional weiteren Pflasterschichten, wobei das Verfahren kontinuierlich oder halbkontinuierlich mit Folienbahnen durchgeführt wird, **dadurch gekennzeichnet, dass** eine wirkstoffhaltige Polyurethan-Trägermatrix (40') auf einer elastischen Trägerfolie (20) erzeugt wird, deren Elastizität größer oder gleich der des ausreagierten Matrix-Polyurethans (40') ist, wobei die elastische Trägerfolie (20) durch eine unterliegende, anhaftende, wenigstens mono-axial nicht-dehnbare Hilfsfolie (30) wenigstens in Verarbeitungsrichtung des Auftrags nicht dehnbar gemacht wurde und eine unmittelbar vor oder mit dem Auftragsvorgang in einer Beschichtungseinheit (60) gemischte lösungsmittelfreie wirkstoffhaltige Polyurethanmasse (40) in einem Reaktivbeschichtungsschritt auf die elastische Trägerfolie (20) beschichtet wird.

2. Verfahren nach Anspruch 1, **dadurch gekennzeichnet, dass** die Folienbahnen aus elastischer Trägerfolie (20) und nicht-dehnbarer Hilfsfolie (30), die der Beschichtungseinheit (60) zugeführt werden, in einem vorgelagerten Verfahrensschritt zu einer Doppelbahn (10) zusammengeführt werden.

3. Verfahren nach Anspruch 1 oder 2, **dadurch gekennzeichnet, dass** die Beschichtungseinheit (60) in einen Schlitz ausläuft und die wirkstoffhaltige nicht ausreagierte Polyurethanmasse (40) in dünner Schicht auf die elastische Folie (20) beschichtet wird.

4. Verfahren nach einem der Ansprüche 1 bis 3, **dadurch gekennzeichnet, dass** die wirkstoffhaltige nicht ausreagierte Polyurethanmasse (40) mit einer Dosier- und Mischmaschine aufgetragen und mit einem Streichmesser auf der elastischen Folie (20) verteilt wird.

5. Verfahren nach einem der Ansprüche 1 bis 4, **dadurch gekennzeichnet, dass** die wirkstoffhaltige nicht ausreagierte Polyurethanmasse (40) mit einer Dosier- und Mischmaschine aufgetragen und durch Aufdrücken einer Walze verteilt wird.

6. Verfahren nach Anspruch 5, **dadurch gekennzeichnet, dass** auf die noch nicht ausreagierte Polyurethanmasse (40) eine zusätzliche Trennfolie aufgelegt und die Masse zwischen Trennfolie und Doppelbahn (10) durch aufdrücken mit einer Walze verteilt wird, wobei die zusätzliche Trennfolie vorzugsweise von der Walze abläuft.

7. Verfahren nach einem der Ansprüche 1 bis 6, **dadurch gekennzeichnet, dass** die Beschichtung in einer Schichtdicke kleiner oder gleich 1000 µm erfolgt.

8. Verfahren nach einem der Ansprüche 1 bis 7, **dadurch gekennzeichnet, dass** innerhalb des reagierenden Polyurethans eine Temperatur von 79 °C nicht überschritten wird.

9. Verfahren nach einem der Ansprüche 1 bis 8, **dadurch gekennzeichnet, dass** die elastische Trägerfolie (20) und die nicht-dehnbare Hilfsfolie (30) in einem Verfahrensschritt nach Fertigstellen der Beschichtung gespalten werden und die nicht-dehnbare Folie (30) von der beschichteten elastischen Folie (20) abgezogen wird.

10. Verfahren nach Anspruch 9, **dadurch gekennzeichnet, dass** anstelle der abgezogenen nicht-dehnbaren Folie (30) wenigstens eine weitere Pflasterschicht aufgebracht wird.

11. Verfahren nach einem der Ansprüche 1 bis 10, **dadurch gekennzeichnet, dass** die beschichtete Folie in einem Verfahrensschritt nach Fertigstellen der Beschichtung und Ausreagieren des Polyurethans geschnitten und dadurch zu Pflasterabschnitten (75) portioniert wird, vorzugsweise einschließlich der unterliegenden nicht-dehnbaren Folie (30) oder der weiteren Pflasterschicht(en).

12. Polyurethanpflasterbahn (70) für die Portionierung zu Einzelpflastern (75), erhältlich mit einem Verfahren nach einem der Ansprüche 1 bis 8, **gekennzeichnet durch** das Vorhandensein folgender Schichten:
- eine für den direkten Hautkontakt vorgesehene lösungsmittelfreie Schicht aus haftklebendem Polyurethan (40') mit definierter Wirkstoffkonzentration je Flächeneinheit, wobei die Bruchdehnung des Polyurethans mehr als 150 % gemessen nach DIN EN ISO 17982008 beträgt,
- eine mit vorgenannter Schicht in Flächenkontakt stehende elastische Trägerfolie (20) deren Elastizität größer oder gleich der des ausreagierten Matrix-Polyurethans (40') ist,
- eine mit der elastischen Trägerfolie (20) in Flächenkontakt stehende, anhaftende, nicht-dehnbare Folie (30), die eine solche Folie ist für deren Längung um 10 % eine Kraft von mehr als 3 Newton je Zentimeter Bahnbreite erforderlich ist,
- und optional eine mit der Schicht aus haftklebendem Polyurethan (40') in Kontakt stehende nicht-dehnbare Trenn- oder Abdeckfolie (90).

13. Polyurethanpflasterbahn (70) nach Anspruch 12, **dadurch gekennzeichnet, dass** die nicht-dehnbare Folie (30) von der elastischen Folie (20) durch Spalten von Hand getrennt werden kann.

14. Polyurethanpflasterbahn (70) nach Anspruch 12, **dadurch gekennzeichnet, dass** die nicht-dehnbare Folie (30) und die elastische Folie (20) einen spaltbaren Verbund bilden, bei dem diese Folien durch punktweise Klebstoffkaschierung verbunden sind oder aus coextrudierten oder kaschierten Lagen einer Mehrschichtfolie bestehen.

## Claims

1. Method for producing a polyurethane patch having a backing film and a layer of a polyurethane matrix and optionally further patch layers, the method being carried out continuously or semi-continuously with film webs, **characterized in that** an active ingredient-containing polyurethane backing matrix (40') is produced on an elastic backing film (20) whose elasticity is greater than or equal to that of the fully reacted matrix polyurethane (40'), the elastic backing film (20) having been made inextensible at least in the processing direction of the application, by means of an underlying, adhering, at least monoaxially inextensible auxiliary film (30), and a solvent-free, active ingredient-containing polyurethane composition (40), mixed immediately beforehand or with the application process in a coating unit (60), is coated onto the elastic backing film (20) in a reactive-coating step.

2. Method according to Claim 1, **characterized in that** the film webs of elastic backing film (20) and inextensible auxiliary film (30), which are supplied to the coating unit (60), are brought together in an upstream method step to form a dual web (10).

3. Method according to Claim 1 or 2, **characterized in that** the coating unit (60) runs out into a slot, and the active ingredient-containing, non-fully-reacted polyurethane composition (40) is coated in a thin layer onto the elastic film (20).

4. Method according to any of Claims 1 to 3, **characterized in that** the active ingredient-containing, non-fully-reacted polyurethane composition (40) is applied with a metering and mixing machine and is spread with a coating blade on the elastic film (20).

5. Method according to any of Claims 1 to 4, **characterized in that** the active ingredient-containing, non-fully-reacted polyurethane composition (40) is applied with a metering and mixing machine and is spread by pressure application of a roll.

6. Method according to Claim 5, **characterized in that** an additional release film is placed onto the as yet not fully-reacted polyurethane composition (40), and the composition is spread between release film and dual web (10) by pressure application with a roll, the additional release film preferably running from the roll.

7. Method according to any of Claims 1 to 6, **characterized in that** the coating takes place in a layer thickness less than or equal to 1000 µm.

8. Method according to any of Claims 1 to 7, **characterized in that** within the reacting polyurethane a temperature of 79°C is not exceeded.

9. Method according to any of Claims 1 to 8, **characterized in that** the elastic backing film (20) and the inextensible auxiliary film (30) are split in a method step following completion of the coating, and the inextensible film (30) is removed from the coated elastic film (20).

10. Method according to Claim 9, **characterized in that**, instead of the inextensible film (30) removed, at least one further patch layer is applied.

11. Method according to any of Claims 1 to 10, **characterized in that** the coated film is cut, in a method step following completion of the coating and full reaction of the polyurethane, and is thereby portioned to form patch sections (75), preferably including the underlying inextensible film (30) or the further patch layer(s).

12. Polyurethane patch web (70) for portioning to individual patches (75), obtainable using a method according to any of Claims 1 to 8, **characterized by** the presence of the following layers:
- a solvent-free layer of pressure-sensitively adhesive polyurethane (40'), intended for direct skin contact, with a defined active ingredient concentration per unit area, the elongation at break of the polyurethane being more than 150% as measured to DIN EN 1S0 17982008,
- an elastic backing film (20) in surface contact with the aforesaid layer whose elasticity is greater than or equal to that of the fully reacted matrix polyurethane (40'),
- an adhering, inextensible film (30) in surface contact with the elastic backing film (20), of a kind requiring a force of more than 3 newtons per centimetre of web width to be lengthened by 10%,
- and optionally an inextensible release or liner film (90) in contact with the layer of pressure-sensitively adhesive polyurethane (40').

13. Polyurethane patch web (70) according to Claim 12, **characterized in that** the inextensible film (30) can be parted from the elastic film (20) by manual splitting.

14. Polyurethane patch web (70) according to Claim 12, **characterized in that** the inextensible film (30) and the elastic film (20) form a splittable assembly in which these films are joined by pointwise lamination with adhesive or consist of coextruded or laminated plies of a multilayer film.

## Revendications

1. Procédé de fabrication d'un timbre en polyuréthane comprenant une feuille support et une couche en une matrice de polyuréthane et éventuellement d'autres couches de timbre, le procédé étant réalisé de manière continue ou semi-continue avec des bandes de feuilles, **caractérisé en ce qu'**une matrice support en polyuréthane contenant un agent actif (40') est formée sur une feuille support élastique (20), dont l'élasticité est supérieure ou égale à celle du polyuréthane de la matrice réagi (40'), la feuille support élastique (20) étant rendue non extensible au moins dans la direction d'usinage de l'application par une feuille auxiliaire (30) sous-jacente adhérente, non extensible au moins dans la direction monoaxiale, et une composition de polyuréthane (40) contenant un agent actif sans solvant mélangée dans une unité de revêtement (60) directement avant ou simultanément avec le processus d'application étant revêtue sur la feuille support élastique (20) lors d'une étape de revêtement réactive.

2. Procédé selon la revendication 1, **caractérisé en ce que** les bandes de feuilles constituées par la feuille support élastique (20) et la feuille auxiliaire non extensible (30), qui sont introduites dans l'unité de revêtement (60), sont réunies en une bande double (10) lors d'une étape de procédé en amont.

3. Procédé selon la revendication 1 ou 2, **caractérisé en ce que** l'unité de revêtement (60) se vide dans une fente et la composition de polyuréthane non réagie contenant un agent actif (40) est revêtue en une couche mine sur la feuille élastique (20).

4. Procédé selon l'une quelconque des revendications 1 à 3, **caractérisé en ce que** la composition de polyuréthane non réagie contenant un agent actif (40) est appliquée avec une machine de dosage et de mélange, et répartie sur la feuille élastique (20) avec un racloir.

5. Procédé selon l'une quelconque des revendications 1 à 4, **caractérisé en ce que** la composition de polyuréthane non réagie contenant un agent actif (40) est appliquée avec une machine de dosage et de mélange, et répartie par application d'un cylindre.

6. Procédé selon la revendication 5, **caractérisé en ce qu'**une feuille de séparation supplémentaire est placée sur la composition de polyuréthane encore non réagie (40), et la composition entre la feuille de séparation et la bande double (10) est répartie par application d'un cylindre, la feuille de séparation supplémentaire étant de préférence déroulée par le cylindre.

7. Procédé selon l'une quelconque des revendications 1 à 6, **caractérisé en ce que** le revêtement a lieu en une épaisseur de couche inférieure ou égale à 1 000 µm.

8. Procédé selon l'une quelconque des revendications 1 à 7, **caractérisé en ce qu'**une température de 79 °C n'est pas dépassée dans le polyuréthane réagissant.

9. Procédé selon l'une quelconque des revendications 1 à 8, **caractérisé en ce que** la feuille support élastique (20) et la feuille auxiliaire non extensible (30) sont séparées lors d'une étape de procédé après la fabrication du revêtement, et la feuille non extensible (30) est ôtée de la feuille élastique revêtue (20).

10. Procédé selon la revendication 9, **caractérisé en ce qu'**au moins une couche de timbre supplémentaire est appliquée à la place de la feuille non extensible ôtée (30).

11. Procédé selon l'une quelconque des revendications 1 à 10, **caractérisé en ce que** la feuille revêtue est découpée lors d'une étape de procédé après la fabrication du revêtement et la réaction du polyuréthane, et ainsi divisée en sections de timbre (75), de préférence incluant la feuille non extensible (30) sous-jacente ou la ou les couches de timbre supplémentaires.

12. Bande de timbre en polyuréthane (70) destinée à être divisée en timbres individuels (75), pouvant être obtenue par un procédé selon l'une quelconque des revendications 1 à 8, **caractérisée par** la présence des couches suivantes :
- une couche sans solvant en polyuréthane adhésif de contact (40'), prévue pour le contact direct avec la peau, contenant une concentration d'agent actif définie par unité de surface, l'allongement à la rupture du polyuréthane étant supérieur à 150 %, mesuré selon DIN EN ISO 17982008,
- une feuille support élastique (20) en contact en surface avec la couche susmentionnée, dont l'élasticité est supérieure ou égale à celle du polyuréthane de la matrice réagi (40'),
- une feuille non extensible adhérente (30) en contact en surface avec la feuille support élastique (20), pour l'allongement de 10 % de laquelle une force de plus de 3 Newton par centimètre de largeur de bande est nécessaire,
- et éventuellement une feuille de séparation ou supérieure non extensible (90) en contact avec la couche en polyuréthane adhésif de contact (40').

13. Bande de timbre en polyuréthane (70) selon la revendication 12, **caractérisée en ce que** la feuille non extensible (30) peut être séparée de la feuille élastique (20) par séparation manuelle.

14. Bande de timbre en polyuréthane (70) selon la revendication 12, **caractérisée en ce que** la feuille non extensible (30) et la feuille élastique (20) forment un composite séparable, dans lequel ces feuilles sont reliées par une pellicule adhésive ponctuelle ou sont constituées de couches coextrudées ou stratifiées d'une feuille multicouche.
